# EUROPEAN PATENT APPLICATION

(11) **EP 3 667 319 A1**
(43) Date of publication of application: **17.06.2020**
(21) Application number: 18844436.8
(22) Date of filing: 17.07.2018
(51) Int. Cl.: G01N 33/53, G01N 33/543, G01N 33/545

(54) **METHOD FOR DIAGNOSING CANINE PREGNANCY AND DIAGNOSTIC REAGENT THEREFOR**

(30) Priority: 09.08.2017 JP 2017154767
(71) Applicant: Yamaguchi University, Yamaguchi 753-8511 (JP); Eiken Kagaku Kabushiki Kaisha, Taito-ku Tokyo 110-8408 (JP)
(72) Inventor: KIMURA Tohru, Yamaguchi-shi Yamaguchi 753-8515 (JP); TOMIYAMA Yurina, Yamaguchi-shi Yamaguchi 753-8515 (JP); ASAHI Yukari, Shimotsuga-gun Tochigi 329-0114 (JP); KIKUCHI Tatsunori, Shimotsuga-gun Tochigi 329-0114 (JP)
(74) Representative: Altmann Stößel Dick Patentanwälte PartG mbB
(86) International application number: PCT/JP2018/026664
(87) International publication number: WO 2019/031163

(57) **Abstract**

It is an object of the present invention to provide a method for diagnosing canine pregnancy and a diagnostic reagent therefor. Specifically, the present invention relates to a method for diagnosing canine pregnancy, comprising a step of immunologically measuring a canine acute phase protein in a biological sample using an anti-canine acute phase protein antibody or a fragment thereof, wherein the canine acute phase protein is not a canine C-reactive protein.

## Description

### Technical Field

The present invention relates to a method for diagnosing canine pregnancy using acute phase protein as an indicator, and a diagnostic reagent therefor.

### Background Art

At present, breeding and production of dogs as companion animals are very active. For breeders, it is important to diagnose the success or failure of the pregnancy after the mating of dogs.

Human pregnancy is diagnosed by measuring human chorionic gonadtropin (hereinafter referred to as "hCG") in urine (or in blood) as a marker. In the case of dogs, however, a marker actually used in the diagnosis of pregnancy, which corresponds to hCG for humans, has not been known.

By the way, acute phase proteins, such as serum amyloid A (hereinafter referred to as "SAA") or a C-reactive protein (hereinafter referred to as "CRP"), have been known as inflammatory markers.

SAA is a precursor protein of amyloid A protein that is deposited in tissue upon amyloidosis, which is a serum protein having a molecular weight of approximately 12000 (Non Patent Literature 1). It has been revealed that the serum value of SAA increases in inflammatory disease other than amyloidosis, and SAA has been evaluated as a sensitive inflammatory marker (Non Patent Literature 2).

CRP has been discovered as a protein having a precipitation reaction with C polysaccharide of pneumococcus. CRP is a protein with a molecular weight of approximately 110000 having a pentamer structure, in which 5 subunits with a molecular weight of approximately 21500 having no sugar chains bind to one another to form a ring, and such CRP has been widely utilized as a marker of inflammatory disease in clinical sites (Non Patent Literature 3).

SAA and CRP have been known to be inflammatory markers that are important also for animal species other than humans (Non Patent Literatures 4 to 9). For example, Patent Literature 1 discloses that the amount of an SAA protein in a milk sample obtained from the udder of lactating mammals (bovines, etc.) clearly correlates with the inflammatory response (mastitis) level of mammary tissues. Patent Literature 2 discloses that infectious etiology can be distinguished from non-infectious etiology based on the concentration of SAA in the blood sample of mammals (horses, etc.) exhibiting abnormal symptoms or behaviors.

Moreover, Patent Literature 3 discloses confirmation of the presence of CRP in canine blood, and that the CRP concentration in serum serves as a marker of inflammatory disease or tissue destruction even in dogs. Patent Literature 4 discloses that the diagnosis of canine inflammatory disease, prognosis judgment thereof, and the diagnosis of postoperative infection can be simply and promptly carried out by measuring canine CRP according to a latex agglutination method using an anti-canine CRP antibody.

On the other hand, Non Patent Literature 10 discloses an increase in serum CRP in pregnant dogs. However, conventionally, it has not been known that canine pregnancy can be diagnosed by using an acute phase protein such as SAA as an indicator.

### Citation List

### Patent Literature

Patent Literature 1: Japanese Patent Publication (Kohyo) No. 2003-507725 A
Patent Literature 2: International Publication No. WO2014/118764
Patent Literature 3: Japanese Patent Publication (Kokai) No. 62-155299 A (1987)
Patent Literature 4: Japanese Patent Publication (Kokai) No. 5-288748 A (1993)

### Non Patent Literature

Non Patent Literature 1: Husby G. and Natvig J.B., J. Clin. Invest., 1974, Vol. 53, pp. 1054-1061
Non Patent Literature 2: Toshiyuki YAMADA et al., Journal of Clinical Laboratory Medicine, 1988, 32:2, pp. 167-172
Non Patent Literature 3: Yoshihisa ITO, Journal of Clinical Laboratory Medicine, 2002, 46:9, pp. 959-966
Non Patent Literature 4: Nunokawa Y. et al., J. Vet. Med. Sci., 1993, 55(6), pp. 1011-1016
Non Patent Literature 5: Kajikawa T. et al., J. Vet. Med. Sci., 1996, 58(11), pp. 1141-1143
Non Patent Literature 6: Tamamoto T. et al., J. Vet. Med. Sci., 2008, 70(11), pp. 1247-1252
Non Patent Literature 7: Petersen H.H. et al., Vet. Res., 2004, 35(2), pp. 163-187
Non Patent Literature 8: Takashi TAMAMOTO, Journal of the Hokkaido Veterinary Medical Association, 2014, 58, pp. 539-543
Non Patent Literature 9: Nakamura M. et al., J. Vet. Med. Sci., 2008, 70(2), pp. 127-131
Non Patent Literature 10: Kuribayashi T. et al., Exp. Anim., 2003, 52(5), pp. 387-390

### Summary of Invention

### Technical Problem

Considering the aforementioned circumstances, it is an object of the present invention to provide a method for diagnosing canine pregnancy and a canine pregnancy diagnostic reagent used in the method.

### Solution to Problem

As a result of intensive studies directed towards achieving the aforementioned object, the present inventors have found that canine pregnancy can be diagnosed using an acute phase protein such as SAA as an indicator, thereby completing the present invention.

Specifically, the present invention includes the following.
(1) A method for diagnosing canine pregnancy, comprising a step of immunologically measuring a canine acute phase protein in a biological sample using an anti-canine acute phase protein antibody or a fragment thereof, wherein the canine acute phase protein is not canine CRP.
(2) The method according to the above (1), wherein the canine acute phase protein is canine SAA.
(3) The method according to the above (2), wherein a dog is determined to be pregnant, when the canine SAA concentration at 20 to 40 days after mating increases in comparison to immediately after mating, or when the canine SAA concentration is 2.0 mg/L or more at 30 days after mating.
(4) The method according to any one of the above (1) to (3), wherein the biological sample is a serum or plasma sample.
(5) The method according to any one of the above (1) to (4), wherein the immunological measurement is a latex turbidimetric immunoassay.
(6) A canine pregnancy diagnostic reagent comprising an anti-canine acute phase protein antibody or a fragment thereof, wherein the canine acute phase protein is not canine CRP.
(7) The reagent according to the above (6), wherein the canine acute phase protein is canine SAA.

This description includes part or all of the content as disclosed in the description and/or drawings of Japanese Patent Application No. 2017-154767, which is a priority document of the present application.

### Advantageous Effects of Invention

According to the present invention, canine pregnancy can be easily diagnosed.

### Brief Description of Drawings

[Figure 1] Figure 1 is a view showing the results obtained by measuring SAA in the serum of dogs after mating, using a latex reagent prepared by immobilizing an anti-canine SAA monoclonal antibody on a polystyrene latex having a particle diameter of 215 nm.
[Figure 2] Figure 2 is a view showing the results obtained by measuring CRP in the serum of dogs after mating, using FUJI DRI-CHEM SLIDE vc-CRP-P (manufactured by FUJIFILM Corporation).

### Description of Embodiments

Hereafter, the present invention will be described in detail.

The method for diagnosing canine pregnancy according to the present invention (hereinafter referred to as "the present method") comprises a step of immunologically measuring a canine acute phase protein in a biological sample using an anti-canine acute phase protein antibody or a fragment thereof. According to the present method, after mating, canine pregnancy can be easily diagnosed (or detected, evaluated, or predicted).

Acute phase protein means a protein that increases in blood at an acute phase of inflammation. The acute phase protein that is used as a measurement target in the present method is not particularly limited, as long as it is an acute phase protein other than CRP. Examples of the acute phase protein may include SAA, fibrinogen, haptoglobin, α1-antitrypsin, α1-antichymotrypsin, and α1-acid glycoprotein. In particular, SAA is preferable.

As described in the Examples below, the peak of SAA increase is concentrated in approximately 30 days after mating. In contrast, the period of CRP increase is over a long period of time from 20 to 50 days after mating. Thus, in comparison to CRP, SAA is able to diagnose (assume) pregnancy by a pin-point measurement.

Moreover, in pregnancy, the SAA level increases at approximately 30 days after mating, and it does not increase in periods other than the aforementioned period, or even if the SAA level increases in other periods, it is only a mild level. Hence, it is possible to distinguish between pregnancy and other inflammatory diseases by measuring SAA at approximately 30 days after mating and at any given time point other than the aforementioned period.

The anti-canine acute phase protein antibody used in the present method may be of any immunoglobulin (Ig) class (IgA, IgG, IgE, IgD, IgM, IgY, etc.) and subclass (IgG1, IgG2, IgG3, IgG4, IgA1, IgA2, etc.). In addition, the light chain of immunoglobulin may be either a κ chain or a λ chain. Examples of the anti-canine acute phase protein antibody may include a polyclonal antibody, a monoclonal antibody, and a recombinant antibody (e.g., a chimeric antibody, a single-chain antibody, etc.).

Moreover, examples of the anti-canine acute phase protein antibody fragment may include Fab, Fab', F(ab')₂, Fv, Fd, and Fabc. A method for producing such fragments is known in the present technical field, and such fragments can be obtained, for example, by digestion of the antibody molecules using protease such as papain or pepsin, or according to a known genetic engineering method.

The biological sample used in the present method may be a biological sample that contains or is likely to contain an acute phase protein. Examples of such a biological sample may include whole blood, serum, plasma, urine, puncture fluid, sweat, saliva, lymph, and cerebrospinal fluid. In particular, serum or plasma is preferable.

In the present method, an anti-canine acute phase protein antibody or a fragment thereof is used to immunologically measure an acute phase protein in a biological sample derived from a dog as a diagnostic target. A canine acute phase protein contained in a biological sample is allowed to come into contact with an anti-canine acute phase protein antibody or a fragment thereof to generate an antigen-antibody reaction, so that the canine acute phase protein in the sample can be detected or measured based on the formed immune complex.

Examples of methods for immunological measurement may include: a single radical immunodiffusion method, which comprises allowing an anti-canine acute phase protein antibody or a fragment thereof to bind with a canine acute phase protein in a biological sample on an agar plate to form an immune complex, and then confirming the expression of a precipitation line due to the immune complex; an enzyme immunoassay and a radioimmunoassay, which use an enzyme- or radioactive isotope-labeled anti-canine acute phase protein antibody or a fragment thereof; and other methods using insoluble carrier particles on each of which an anti-canine acute phase protein antibody or a fragment thereof is immobilized. Examples of the insoluble carrier particles may include latex particles such as polyethylene or polystyrene particles, alumina particles, silica particles, colloidal gold, and magnetic particles. Among these insoluble carrier particles, latex particles, and in particular, polystyrene latex particles are preferably used.

Furthermore, the particle diameter of an insoluble carrier particle is preferably 50 to 500 nm, and more preferably 75 to 350 nm.

Further, as a method of immobilizing an anti-canine acute phase protein antibody or a fragment thereof on an insoluble carrier particle, there can be applied a known technique, namely, a method comprising mixing the antibody or a fragment thereof with an insoluble carrier particle to perform physical adsorption of the antibody or a fragment thereof on the surface of the insoluble carrier particle, so that the antibody or a fragment thereof can be immobilized on the insoluble carrier particle.

Further, in the case of using an insoluble carrier particle into the surface of which an amino group or a carboxyl group is introduced, the antibody or a fragment thereof can be immobilized on the surface of the insoluble carrier particle by a covalent bond using a glutaraldehyde or carbodiimide reagent.

As a method of using an anti-canine acute phase protein antibody or a fragment thereof labeled with an enzyme or a radioactive isotope, for example, an enzyme immunoassay of using a microplate, on which an anti-canine acute phase protein antibody or a fragment thereof is immobilized, and an enzyme-labeled anti-canine acute phase protein antibody or a fragment thereof (secondary antibody), may be applied. Specifically, in such an enzyme immunoassay, the anti-canine acute phase protein antibody or a fragment thereof that has been immobilized on the microplate is allowed to react with a canine acute phase protein in a biological sample, and further, an enzyme-labeled anti-canine acute phase protein antibody or a fragment thereof (secondary antibody) is added to the reaction mixture, so that they are allowed to react with each other. Thereafter, the thus obtained reaction mixture is washed and is then allowed to react with an enzyme substrate, so as to measure the canine acute phase protein based on coloration caused by the enzyme reaction.

Examples of the method of using an insoluble carrier particle may include: a latex turbidimetric immunoassay of using latex particles formed by immobilizing an anti-canine acute phase protein antibody or a fragment thereof on latex; a colloidal gold aggregation colorimetric method of using colloidal gold particles formed by immobilizing an anti-canine acute phase protein antibody or a fragment thereof on colloidal gold particle; and an immunochromatography method, in which an anti-canine acute phase protein antibody or a fragment thereof labeled with colloidal metal particle, etc., and a capture antibody that captures an immune complex of the anti-canine acute phase protein antibody or a fragment thereof and a canine acute phase protein, are used on a membrane such as a nitrocellulose membrane. Specifically, in the latex turbidimetric immunoassay, latex particles formed by immobilizing an anti-canine acute phase protein antibody or a fragment thereof on latex are allowed to react with a canine acute phase protein in a biological sample to form an immune complex, and as a result of the formation of the immune complex, the latex particles agglutinate, and then, the canine acute phase protein is measured based on a change in turbidity caused by the agglutination of the latex particle. On the other hand, in the immunochromatography method, on a membrane such as a nitrocellulose membrane, a biological sample is supplied, and a canine acute phase protein in the biological sample is allowed to react with an anti-canine acute phase protein antibody or a fragment thereof labeled with colloidal metal, etc., in a labeling reagent-retaining part that retains the anti-canine acute phase protein antibody or a fragment thereof labeled with colloidal metal particle, etc., so as to form an immune complex, and the immune complex moves in the membrane by a capillary phenomenon, and is then captured by a capture antibody immobilized on a predetermined position of the membrane, so that the canine acute phase protein can be detected based on coloration caused by the capturing.

Meanwhile, a calibration curve is prepared from the measurement results of a canine acute phase protein standards/calibrators. Based on the calibration curve, the concentration of the canine acute phase protein in the biological sample can be determined from absorbance or the like measured by the aforementioned methods for immunological measurement.

Based on the measured concentration of the canine acute phase protein in the biological sample, whether a dog from which the biological sample is derived is pregnant or not can be diagnosed.

For example, when the canine SAA concentration in a diagnostic target dog-derived biological sample at 20 to 40 days (preferably at approximately 30 days) after mating increases, in comparison to the canine SAA concentration in the diagnostic target dog-derived biological sample immediately after mating, or when the canine SAA concentration in the diagnostic target dog-derived biological sample at 20 to 40 days (preferably at approximately 30 days) after mating is 2 mg/L or more, preferably 3 mg/L or more, and more preferably 4.0 mg/L or more, the target dog can be determined to be pregnant.

The canine pregnancy diagnostic reagent (or kit) according to the present invention (hereinafter referred to as "the reagent according to the present invention") comprises an anti-canine acute phase protein antibody or a fragment thereof. The reagent according to the present invention is used in a method for immunological measurement performed on a canine acute phase protein in the present method. For instance, when the method for immunological measurement is a method using an insoluble carrier particle, the reagent according to the present invention may comprise an insoluble carrier particle on which an anti-canine acute phase protein antibody or a fragment thereof is immobilized, for example, a latex solution containing latex particle. When the method for immunological measurement is an immunochromatography method, the reagent according to the present invention may be used in an immunochromatographic device including an insoluble carrier particle on which an anti-canine acute phase protein antibody or a fragment thereof is immobilized, such as colloidal gold particle, and a membrane such as a nitrocellulose membrane, on which an anti-canine acute phase protein antibody or a fragment thereof is immobilized (e.g., a membrane such as a nitrocellulose membrane, supported on a supporting body, having a sample-supplying part, a labeling reagent-retaining part that retains an anti-canine acute phase protein antibody or a fragment thereof labeled with colloidal metal, etc., and a detection part including a capture antibody immobilized on a predetermined position).

Moreover, the reagent according to the present invention may comprise a buffer that gives a pH value necessary for immunological reaction, a reaction enhancer that promotes immunological reaction, a reaction stabilizer or a blocker that suppresses non-specific reactions, an antiseptic enhancing the preservation properties of the reagent, such as sodium azide, in addition to an anti-canine acute phase protein antibody or a fragment thereof.

Examples of the buffer may include the following buffers.

Good's buffers:
2-(N-morpholino)ethanesulfonic acid (which is abbreviated as "MES");
piperazine-N,N'-bis(2-ethanesulfonic acid) (which is abbreviated as "PIPES");
N-(2-acetamido)-2-aminoethanesulfonic acid (which is abbreviated as "ACES");
N,N-bis(2-hydroxyethyl)-2-aminoethanesulfonic acid (which is abbreviated as "BES");
bis(2-hydroxyethyl)iminotris(hydroxymethyl)methane (which is abbreviated as "Bis- Tris");
3-[N,N-bis(2-hydroxyethyl)amino]-2-hydroxypropanesulfonic acid (which is abbreviated as "DIPSO");
N-2-hydroxyethylpiperazine-N'-3-propanesulfonic acid (which is abbreviated as "EPPS");
N-2-hydroxyethylpiperazine-N'-2-ethanesulfonic acid (which is abbreviated as "HEPES");
N-2-hydroxyethylpiperazine-N'-2-hydroxypropane-3-sulfonic acid (which is abbreviated as "HEPPSO");
3-(N-morpholino)propanesulfonic acid (which is abbreviated as "MOPS");
3-(N-morpholino)-2-hydroxypropanesulfonic acid (which is abbreviated as "MOPSO");
piperazine-N,N'-bis(2-hydroxypropanesulfonic acid) (which is abbreviated as "POPSO");
N-tris(hydroxymethyl)methyl-3-aminopropanesulfonic acid (which is abbreviated as "TAPS");
N-tris(hydroxymethyl)methyl-2-hydroxy-3-aminopropanesulfonic acid (which is abbreviated as "TAPSO"); and
N-tris(hydroxymethyl)methyl-2-aminoethanesulfonic acid (which is abbreviated as "TES").

Other buffers:
2-amino-2-hydroxymethyl-1,3-propanediol (which is also referred to as "tris(hydroxymethyl)aminomethane");
phosphate buffer; and
ammonium buffer.

Among these buffers, a Good's buffer such as HEPES or PIPES is particularly preferable because it does not only give a pH value that is advantageous for immunological reaction, but it also has a small influence on the protein. The pH value necessary for immunological reaction may be pH 5 to 11, and may be preferably pH 6 to 9.

As reaction enhancers, polyethylene glycol, dextran sulfate and the like have been known. Further, examples of the reaction stabilizer or the blocker may include BSA (bovine serum albumin), animal serum, IgG, IgG fragments (Fab or Fc), albumin, lactoprotein, amino acid, polyamino acid, choline, polysaccharides such as sucrose, gelatin, gelatin degradation products, casein, and polyhydric alcohol such as glycerin. These reaction stabilizers or blockers have been known to be effective for stabilization of reaction or suppression of non-specific reactions in immunological reaction.

The reagent according to the present invention comprising various types of components, as described above, can be supplied in the form of a solution or in a dried condition. In order to distribute the present reagent in the form of a solution, various types of surfactants, sugars, inactive proteins, etc. may be further added to the reagent for the purpose of enhancing the stability of the protein. These stabilizers are also effective as stabilizers or excipients, when the present reagent is dried.

### Examples

Hereinafter, the present invention will be more specifically described in the following Examples. However, these Examples are not intended to limit the technical scope of the present invention.

### [Example 1] Measurement of canine SAA concentration at 1 to 60 days after mating

### 1. Purpose

The SAA concentration in the serum of 15 dogs at 1, 10, 20, 30, 40, 50, and 60 days after mating, or the SAA concentration in the serum of 5 dogs at 30 days after mating, was measured, and whether the diagnosis of pregnancy can be made was confirmed.

### 2. Materials and Methods

SAA in a canine serum specimen after mating was measured using a latex reagent immobilizing an anti-canine SAA monoclonal antibody.

The above-described latex reagent was produced using an anti-canine SAA monoclonal antibody and a polystyrene latex (particle diameter: 215nm).

The anti-canine SAA monoclonal antibody was immobilized on the above-described polystyrene latex particles according to a known method. Specifically, immobilization of the anti-canine SAA monoclonal antibody was carried out by mixing the anti-canine SAA monoclonal antibody with the polystyrene latex particles, and then physically adsorbing the anti-canine SAA monoclonal antibody on the surface of the polystyrene latex particles.

The aforementioned measurement of the canine serum specimen after mating was carried out using Hitachi 7170S Clinical Analyzer (manufactured by Hitachi High-Technologies Corporation). As a measurement method, 80 µL of a first reagent (a 50 mM HEPES buffer, pH 7.4) was mixed with 2.4 µL of a serum specimen, followed by performing incubation at 37°C for 5 minutes. Thereafter, 80 µL of a second reagent (an anti-canine SAA monoclonal antibody-immobilizing latex solution comprising a 10 mM HEPES buffer (pH 7.4)) was mixed with the above-mixed solution, so that a reaction was carried out at 37°C. Approximately 5 minutes after addition of the second reagent, a change in the absorbance at a wavelength of 660 nm was measured.

The SAA concentration in the canine serum specimen after mating was calculated from a calibration curve obtained by measuring samples each having a known SAA concentration.

### 3. Results

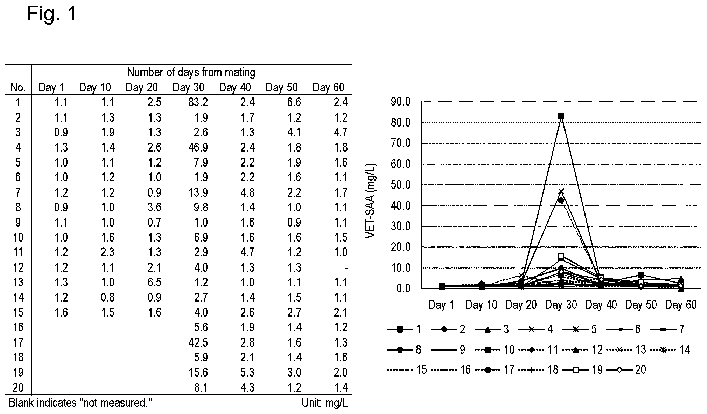

The results are shown in Figure 1. As shown in Figure 1, an increase in the SAA concentration in the serum specimen was observed at 20, 30, or 40 days after mating, in comparison to immediately after mating (on a first day of mating). In particular, at 30 days after mating, there were a large number of individual dogs, in which an increase in the SAA concentration was observed.

### 4. Conclusion

It is possible to diagnose canine pregnancy after mating from the comparison in terms of the SAA concentration in the serum specimen between immediately after mating and at 20 to 40 days after mating, or the SAA concentration at 20 to 40 days after mating. In particular, the SAA concentration at 30 days after mating is useful.

### [Comparative Example 1] Measurement of canine CRP concentration at 1 to 60 days after mating

### 1. Purpose

The CRP concentration in the serum of 15 dogs at 1, 10, 20, 30, 40, 50, and 60 days after mating, or the CRP concentration in the serum of 5 dogs at 30 days after mating, was measured, and whether the diagnosis of pregnancy can be made was confirmed. The canine serum specimen as subjected to the measurement is the same specimen as that measured in Example 1.

### 2. Materials and Methods

CRP in the canine serum specimen after mating was measured using FUJI DRI-CHEM SLIDE vc-CRP-P (manufactured by FUJIFILM Corporation).

The measurement of the canine serum specimen after mating using the above-described FUJI DRI-CHEM SLIDE vc-CRP-P was carried out by employing FUJI DRI-CHEM (manufactured by FUJIFILM Corporation).

### 3. Results

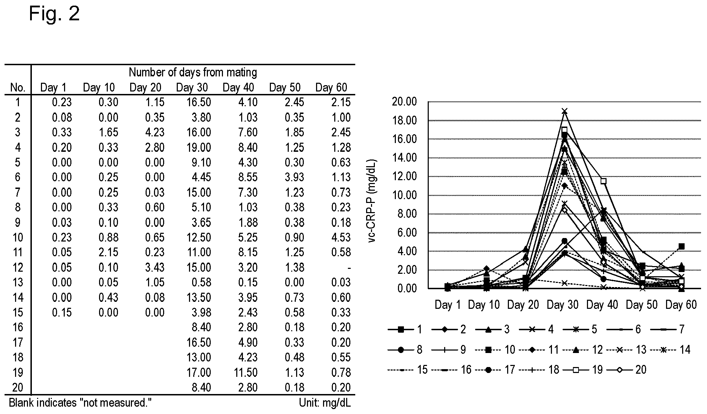

The results are shown in Figure 2. As shown in Figure 2, an increase in the CRP concentration in the serum specimen was observed at 20 to 50 days after mating, in comparison to immediately after mating (on a first day of mating).

### 4. Conclusion

It is possible to diagnose canine pregnancy after mating from the comparison in terms of the CRP concentration in the serum specimen between immediately after mating and at 20 to 50 days after mating, or the CRP concentration at 20 to 50 days after mating.

However, in the case of SAA shown in Example 1, the period, in which an increase in the acute phase protein due to pregnancy is observed after mating, is concentrated in approximately 30 days after mating. Other than this period, an increase in the acute phase protein is not observed, or only a mild increase is observed. In contrast, in the case of CRP, the period of CRP increase is over a long period of time from 20 to 50 days after mating. Thus, in comparison to CRP, SAA can be used to diagnose (assume) pregnancy by a pin-point measurement.

All publications, patents and patent applications cited in the present description are incorporated herein by reference in their entirety.

## Claims

1. A method for diagnosing canine pregnancy, comprising a step of immunologically measuring a canine acute phase protein in a biological sample using an anti-canine acute phase protein antibody or a fragment thereof, wherein the canine acute phase protein is not a canine C-reactive protein.

2. The method according to claim 1, wherein the canine acute phase protein is canine serum amyloid A.

3. The method according to claim 2, wherein a dog is determined to be pregnant, when the canine serum amyloid A concentration at 20 to 40 days after mating increases in comparison to immediately after mating, or when the canine serum amyloid A concentration is 2.0 mg/L or more at 30 days after mating.

4. The method according to any one of claims 1 to 3, wherein the biological sample is a serum or plasma sample.

5. The method according to any one of claims 1 to 4, wherein the immunological measurement is a latex turbidimetric immunoassay.

6. A canine pregnancy diagnostic reagent comprising an anti-canine acute phase protein antibody or a fragment thereof, wherein the canine acute phase protein is not a canine C-reactive protein.

7. The reagent according to claim 6, wherein the canine acute phase protein is canine serum amyloid A.
